# EUROPÄISCHE PATENTSCHRIFT

(11) **EP 2 322 494 B1**
(45) Veröffentlichungstag und Bekanntmachung des Hinweises auf die Patenterteilung: **09.01.2013**
(21) Anmeldenummer: 09014332.2
(22) Anmeldetag: 17.11.2009
(51) Int. Cl.: C07C 41/09, C07C 43/04, C07C 41/42

(54) **Herstellung von Dimethylether aus Rohmethanol**
Production of dimethyl ether from crude methanol
Fabrication de diméthyléther à partir de méthanol brut

(43) Veröffentlichungstag der Anmeldung: 18.05.2011
(73) Patentinhaber: Lurgi GmbH, 60439 Frankfurt am Main (DE)
(72) Erfinder: Mitschke, Peter, 63477 Maintal (DE); Dr. Seidel, Eckhard, 60389 Frankfurt am Main (DE); Renner, Thomas, 60318 Frankfurt am Main (DE); Dr. Rothaemel, Martin, 60437 Frankfurt am Main (DE)

(56) Entgegenhaltungen:
- EP-A2- 0 336 378
- WO-A1-2006/113293
- US-A- 2 014 408
- US-A- 4 149 940
- US-B1- 6 740 783

## Beschreibung

### Gebiet der Erfindung

Die Erfindung betrifft die Herstellung von Dimethylether aus Rohmethanol. Insbesondere betrifft die Erfindung ein Verfahren zur Herstellung von Dimethylether durch katalytische Dehydratisierung von Rohmethanol in der Gasphase sowie einen Einsatz, bei dessen Verwendung ein stabiler Langzeitbetrieb des erfindungsgemäßen Verfahrens sichergestellt werden kann.

### Stand der Technik

Die katalytische Herstellung von Dimethylether (DME) aus Methanol durch katalytische Dehydratisierung ist seit vielen Jahren bekannt. So beschreibt das US-Patent US 2014408 ein Verfahren zur Herstellung und Reinigung von DME aus Methanol an Katalysatoren wie Aluminiumoxid, Titanoxid und Bariumoxid, wobei Temperaturen von 350 bis 400°C bevorzugt sind.

Weitere Informationen zum Stand der Technik und zur heutigen Praxis der Herstellung von Dimethylether finden sich in Ullmann's Encyclopedia of Industrial Chemistry, Sixth Edition, 1998 Electronic Release, Stichwort "Dimethyl Ether". Insbesondere wird dort in Kapitel 3 "Production" dargelegt, dass die katalytische Umsetzung von reinem, gasförmigem Methanol in einem Festbettreaktor durchgeführt wird, und das Reaktionsprodukt nach zweistufiger Kondensation dann einer Destillation zugeführt wird, in der das DME-Produkt von einer Methanol-Wasser-Mischung abgetrennt wird. Die Methanol-Wassermischung wird dann in einer zweiten Kolonne aufgetrennt, wobei das Wasser dem Prozess entzogen und das Methanol wieder in den DME-Reaktor zurückgeführt wird.

Hervorzuheben ist, dass die bisherige industrielle Praxis darin besteht, Reinmethanol zur Herstellung von DME einzusetzen, wie es von Vishwanathan et al., Applied Catalysis A: General 276 (2004) 251-255 ausgeführt wird. Unter Reinmethanol versteht man dabei ein aufgereinigtes, weitgehend wasserfreies Produkt der Methanolsynthese. Das direkte Produkt der Methanolsynthese wird dagegen als Rohmethanol bezeichnet und enthält neben mehreren Gew.-% Wasser auch höhere Alkohole, Ether, Ester, Ketone, Aldehyde, Kohlenwasserstoffe und gelöste Synthesegasbestandteile jeweils in Spuren (Ullmann's Encyclopedia of Industrial Chemistry, Sixth Edition, 1998 Electronic Release, Stichwort "Methanol", Kap. 4.1.3 "Byproducts").

Die Herstellung von Reinmethanol aus dem direkten Produkt der Methanolsynthese, dem Rohmethanol, erfolgt in der Regel durch mehrstufige Destillation oder Rektifikation, wobei im ersten Schritt in einer sog. Leichtsiederkolonne die Bestandteile mit niedrigerem Siedepunkt als Methanol als Kopfprodukte abgetrennt werden; dieses Zwischenprodukt wird auch im Hinblick auf die Entfernung gelöster Gase als stabilisiertes Rohmethanol bezeichnet. Gelegentlich erfolgt auch zunächst eine destillative Teilabtrennung von Wasser, wobei auch das dabei erhaltene Methanol-Produkt noch als Rohmethanol bezeichnet wird. Anschließend wird in mindestens einer weiteren Destillation weitgehend wasserfreies Reinmethanol als Kopfprodukt gewonnen (Ullmann's Encyclopedia of Industrial Chemistry, Sixth Edition, 1998 Electronic Release, Stichwort "Methanol", Kap. 5.4 "Distillation of Crude Methanol").

Die Herstellung von Reinmethanol aus Rohmethanol ist sowohl apparativ als auch energetisch aufwendig, da in der Reinmethanolkolonne große Mengen des Leichtsieders Methanol von kleineren Mengen des Schwersieders Wasser abgetrennt werden müssen. Für die Anwendung von Reinmethanol in der nachfolgenden DME-Herstellung stellt dies eine ökonomische Belastung dar, da das Methanol vollständig erneut verdampft werden muss. Es besteht daher seit längerer Zeit das Bedürfnis, ein praxistaugliches Verfahren zur Herstellung von DME ausgehend von Rohmethanol zur Verfügung zu stellen. So lehrt und beansprucht die deutsche Offenlegungsschrift DE 3817816 A1 ein in eine Methanolsynthese integriertes Verfahren zur katalytischen Herstellung von DME aus Methanol über Dehydratisierungskatalysatoren, das dadurch gekennzeichnet ist, dass das aus dem Methanolsynthesereaktor austretende Gemisch zumindest teilweise, ohne vorherige Abtrennung des nicht umgesetzten Synthesegases und ohne Reinigung des erzeugten Methanols, in einem Dehydratisierungsreaktor an einem geeigneten Katalysator, bevorzugt γ-Al₂O₃, zur Gewinnung von DME umgesetzt wird.

Die US-Patentschrift US 6740783 B1 beschreibt ein Verfahren zur Herstellung von DME aus Rohmethanol. Hier wird ausgeführt, dass bei Verwendung üblicher Dehydratisierungskatalysatoren auf Aluminiumoxid-Basis die Aktivität des Katalysators durch den Wassergehalt im Rohmethanol beeinträchtigt wird. Als Lösung wird vorgeschlagen, als Dehydratisierungskatalysator einen hydrophoben Zeolith zu verwenden, der in Gegenwart von Wasser weniger stark desaktiviert. Zudem soll die Bindung von Wasser an stark Lewis-acide Zentren des Zeolithkatalysators die Verkokung des Katalysators unterdrücken.

Einen ähnlichen Lösungsweg beschreiten die Erfinder in der US-Patentanmeldung US 2009/0023958 A1. Wiederum liegt der Erfindung die Aufgabe zu Grunde, ein Verfahren zur katalytischen Dehydratisierung von Rohmethanol in der Gasphase zur Verfügung zu stellen. Gelöst wird diese Aufgabe nach Angabe der Erfinder dadurch, dass der Rohmethanol-Einsatzstrom zuerst über einen metalldotierten, hydrophoben Zeolithkatalysator und nachfolgend über einen Katalysator ausgewählt aus γ-Al₂O₃ oder SiO₂/Al₂O₃ geleitet wird, wobei die Dehydratisierungsreaktion in einem adiabaten Reaktor durchgeführt wird. Diese Kombination an Verfahrensmerkmalen soll nach Angaben der Erfinder Vorteile bezüglich der Temperaturführung im Reaktor, der geringen Nebenproduktbildung sowie der geringeren Katalysatordesaktivierung aufweisen.

Insgesamt ist daher festzustellen, dass im Stand der Technik bereits verschiedene Verfahren bzw. Verfahrensvarianten zur Herstellung von Dimethylether durch katalytische Dehydratisierung von Rohmethanol in der Gasphase vorgeschlagen werden, sich die vorgeschlagenen Verfahren in der industriellen Praxis aber nicht durchgesetzt haben. Trotz des oben diskutierten, einschlägigen Stands der Technik arbeiten heute alle technischen Anlagen zur Herstellung von Dimethylether durch katalytische Dehydratisierung von Methanol in der Gasphase weiterhin unter Einsatz von Reinmethanol als Einsatz. Trotz der beschriebenen ökonomischen Vorteile scheinen daher grundsätzliche Nachteile bei der Verwendung von Rohmethanol als Einsatz zur bestehen, die bislang nicht überwunden werden konnten.

### Beschreibung der Erfindung

Die Aufgabe der vorliegenden Erfindung besteht daher darin, ein Verfahren zur Herstellung von Dimethylether durch katalytische Dehydratisierung von Rohmethanol in der Gasphase zur Verfügung zu stellen, das die genannten Nachteile vermeidet, und das für den industriellen Einsatz geeignet ist.

Die vorstehend genannte Aufgabe wird mit der Erfindung durch ein Verfahren zur Herstellung von Dimethylether durch katalytische Dehydratisierung von Rohmethanol in der Gasphase gelöst, das folgende Verfahrensschritte umfasst:
(a) Bereitstellen von Rohmethanol aus der Niederdruck-Methanolsynthese,
(b) Verdampfen des Rohmethanols, ggf. nach vorheriger Stabilisierung und/oder nach Wasser-Teilabtrennung und Einstellen einer Reaktionstemperatur und eines Reaktionsdruckes,
(c) Beaufschlagen eines mit Dehydratisierungskatalysator gefüllten Reaktors mit dem verdampften Rohmethanol mit definierter Raumgeschwindigkeit,
(d) Ableiten eines gasförmigen Produktgemisches, umfassend Dimethylether, nicht umgesetztes Methanol und Wasser,
(e) Abkühlen, Teilkondensation und Auftrennung des gasförmigen Produktgemisches, wobei als Produkte gasförmiger Dimethylether sowie flüssiges Wasser und Methanol erhalten werden, wobei das Methanol zum Verfahrensschritt 1 (a) zurückgeführt wird, und das dadurch gekennzeichnet ist, dass das als Einsatz verwendete Rohmethanol einen Gesamtgehalt an Carbonylverbindungen von höchstens 100 Gew.-ppm, bevorzugt von höchstens 50 Gew.-ppm, gerechnet als Massenäquivalente Aceton, aufweist.

Es wurde gefunden, dass bei der Herstellung von Dimethylether durch katalytische Dehydratisierung von Rohmethanol in der Gasphase der Gehalt an Carbonylverbindungen im Rohmethanol eine entscheidende Bedeutung für die Langzeitbeständigkeit des Verfahrens besitzt. Dies ist überraschend, da die negativen Auswirkungen sauerstoffhaltiger Spurenkomponenten auf die Durchführung des Herstellungsverfahrens oder die hierfür verwendete Anlage bei der Herstellung von DME ausgehend von Rohmethanol im Stand der Technik bislang nicht thematisiert oder sogar verneint wurden. So lehrt die internationale Patentanmeldung WO 01/21561 A1, dass bei der über das Zwischenprodukt DME verlaufenden Herstellung kurzkettiger Olefine aus Methanol die Anwesenheit organischer, sauerstoffhaltiger Spurenkomponenten wie höherer Alkohole, Aldehyde oder anderer oxygenierter Verbindungen nur unwesentlichen Einfluss auf die Reaktion hat. Im Gegensatz dazu wurde nun festgestellt, dass beim Überschreiten eines Grenzwertes von 100 Gew.-ppm für den Gesamtgehalt an Carbonylverbindungen, gerechnet als Massenäquivalente Aceton, im Rohmethanol-Einsatz eine Vielzahl zusätzlicher Spurenkomponenten im DME-Produkt auftritt, die als Verunreinigung unerwünscht sind. Dies gilt insbesondere für den Fall, dass nur das Aceton als Carbonylverbindung im Rohmethanol enthalten ist. Enthält der Rohmethanol-Einsatz dagegen auch höhere, potentiell reaktivere Carbonylverbindungen wie Methylethylketon (MEK), wird ein Gesamtgehalt an Carbonylverbindungen im Rohmethanol von höchstens 50 Gew.-ppm bevorzugt, da beobachtet wurde, dass beim Einhalten dieses Grenzwertes keine unbekannten, potentiell schädlichen Spurenkomponenten im DME-Produkt auftreten.

Zudem wurde gefunden, dass diese Spurenkomponenten aufgrund von Kondensations-oder Polymerisationsreaktionen feste Produkte bilden, die zur Bildung von Ablagerungen innerhalb der Anlage und/oder auf dem Katalysator führen, wodurch ein Verstopfen von Anlagenteilen wie Wärmetauschern bzw. die vorzeitige Desaktivierung des Katalysators resultiert. Solche Ablagerungen wurden in entsprechenden, unten beschriebenen Versuchen beobachtet. Als bedeutender Inhaltsstoff der Ablagerungen konnte mittels analytischer Bestimmung Hexamethylbenzol (HMB) identifiziert werden. Dieses entsteht in an sich bekannter Weise aus der Reaktion von Methanol mit Aceton und führt aufgrund seines hohen Schmelzpunktes von 165 °C zur Bildung fester Ablagerungen in kälteren Anlagenteilen sowie zur Verkokung des Katalysators. Beschrieben wird diese Reaktion von Jayamani et al., Indian Journal of Chemistry, Section B: Organic Chemistry Including Medicinal Chemistry (1985), 24B(6), 687-9, zur präparativen Herstellung von HMB. Auch Ganesan und Pillai beschreiben im Journal of Catalysis 119, 288 - 299 (1989) die Umsetzung von Methanol mit unterschiedlichen Ketonen und Aldehyden an einem Al₂O₃-Katalysator zu Hexamethylbenzol (HMB), wobei bei 350°C Aceton und MEK zu 100% umgesetzt werden und HMB mit Ausbeuten von 87 bis 90% entsteht. Mechanistisch gesehen soll die Reaktion dabei - unabhängig von der Art der Carbonylverbindung - stets über Aceton verlaufen, so dass Aceton als sinnvolle Bezugskomponente für die Angabe des Gesamtgehalts an Carbonylverbindungen erscheint. Dies ist von besonderem Interesse, da Rohmethanol diese Verbindungen enthält und bei der DME-Herstellung gemäß Gasphasenverfahren ebenfalls Al₂O₃ als Katalysator einsetzt wird. Folglich können die unerwünschten Kondensationsreaktionen zu hochsiedenden Verbindungen wie HMB nicht nur unter Beteiligung von Aceton, sondern auch in Anwesenheit anderer Carbonylverbindungen ablaufen. Es ist allerdings zu berücksichtigen, dass bei den in der Arbeit von Ganesan und Pillai beschriebenen Versuchen immer sehr hohe Konzentrationen der Carbonylverbindungen von ca. 16 mol-% eingesetzt wurden, was deutlich über den üblichen Konzentrationen dieser Verbindungen im Rohmethanol liegt, die nur einige zehn bis einige hundert ppm betragen.

Überraschenderweise zeigte sich, dass sich Grenzwerte für tolerierbare Mengen von Carbonylverbindungen im Rohmethanol definieren lassen, bei deren Einhalten ein stabiler Langzeitbetrieb der DME-Herstellungsanlage möglich ist und keine Verunreinigungen im DME-Produkt in störender Konzentration nachgewiesen werden. Es wurde gefunden, dass für einen Gesamtgehalt an Carbonylverbindungen von höchstens 100 Gew.-ppm, gerechnet als Massenäquivalent Aceton, die Nebenreaktionen in so untergeordnetem Maße ablaufen, dass der Anlagenbetrieb und der Katalysator nicht negativ beeinflusst werden. Dies gilt insbesondere für den Fall, dass nur das Aceton im Rohmethanol enthalten ist. Enthält der Rohmethanol-Einsatz dagegen auch höhere, potentiell reaktivere Carbonylverbindungen wie Methylethylketon (MEK), wird ein Gesamtgehalt an Carbonylverbindungen im Rohmethanol von höchstens 50 Gew.-ppm, gerechnet als Massenäquivalent Aceton, bevorzugt, da beobachtet wurde, dass beim Einhalten dieses Grenzwertes keine unbekannten, potentiell schädlichen Spurenkomponenten im DME-Produkt auftreten. Dementsprechend lassen sich entsprechende Grenzwerte für ein als Einsatz für die DME-Herstellung bestimmtes Rohmethanol angeben, bei deren Einhaltung noch ein störungsfreier Betrieb der Anlage möglich ist, und ein ausreichend reines DME-Produkt erhalten wird.

Bei der Herstellung von Dimethylether durch katalytische Dehydratisierung von Reinmethanol in der Gasphase tritt der genannte Effekt nicht auf, da der Gesamtgehalt an Carbonylverbindungen in Reinmethanol sehr niedrig ist, wobei üblicherweise nur der Acetongehalt ausgewiesen wird. So weist Reinmethanol der Reinheitsstufe "Grade AA" einen Acetongehalt unter 20 Gew.-ppm auf (Supp, E., How to Produce Methanol from Coal, Springer Verlag, Berlin (1989), S. 134). Eine aktuellere Referenzspezifikation der International Methanol Producers and Consumers Association nennt einen Aceton-Grenzwert von 30 mg/kg (Januar 2008, http://www.impca.be/).

Es wird vermutet, dass die Problematik der Anwesenheit sauerstoffhaltiger, organischer Spurenkomponenten bei früheren Arbeiten zur katalytischen Dehydratisierung von Rohmethanol in der Gasphase zu DME nicht ausreichend berücksichtigt wurde, da dort das Augenmerk auf den Wassergehalt des Rohmethanols gerichtet war. Möglicherweise wurde bei vielen der im Stand der Technik beschriebenen Untersuchungen aus den Reinchemikalien Methanol und Wasser zusammengemischtes, synthetisches Rohmethanol anstelle von anlagenstämmigem Rohmethanol aus einer technischen Anlage zur Methanolsynthese eingesetzt, so dass die genannte Problematik nicht erkannt werden konnte.

Die US-Patentschrift US 4560807 erwähnt die Möglichkeit, neben reinem Methanol auch ein nicht weiter spezifiziertes Nebenprodukt-Methanol mit einem höheren Anteil von anderen Oxygenaten als Rohstoff für die DME-Herstellung einzusetzen. Genannt werden in diesem Zusammenhang die Verbindungen Methylethylether, Methylformiat und Formal (Dimethoxymethan). Allerdings beziehen sich die Angaben lediglich auf zu erwartende Anreicherungen dieser Verunreinigungen im DME-Produkt und nicht auf deren möglicherweise schädlichen Auswirkungen auf die Durchführung des Herstellungsverfahrens oder die hierfür verwendete Anlage selbst. In dem in der Patentschrift enthaltenen Zahlenbeispiel wird wiederum nur Reinmethanol verwendet.

### Bevorzugte Ausgestaltungen der Erfindung

Besonders bevorzugt wird als Reaktor ein Festbettreaktor verwendet. Dieser Reaktortyp zeichnet sich durch seine konstruktive Einfachheit aus und hat sich bei der Herstellung von DME ausgehend von Reinmethanol bewährt.

Eine vorteilhafte Ausgestaltung des erfindungsgemäßen Verfahrens sieht vor, als Katalysator y-Al₂O₃ zu verwenden. Auch andere acide Festkatalysatoren können in dem erfindungsgemäßen Verfahren eingesetzt werden, jedoch weist y-Al₂O₃ Vorteile bezüglich seiner Handhabung, seiner geringen Toxizität sowie ökonomische Vorteile auf.

Bevorzugt liegt die Reaktionstemperatur bei dem erfindungsgemäßen Verfahren zwischen 200 und 500 °C, besonders bevorzugt zwischen 250 und 450 °C. Der Reaktionsdruck liegt bevorzugt zwischen 1 und 100 bar(a), besonders bevorzugt zwischen 1 und 30 bar(a). Als geeignete Raumgeschwindigkeiten haben sich Werte zwischen 1 und 8 kg/(kg h), bevorzugt zwischen 1 und 6 kg/(kg h) erwiesen. Die Raumgeschwindigkeit wird dabei definiert als kg Methanol pro h und pro kg Katalysator.

Vorteilhaft ist es, wenn stabilisiertes Rohmethanol als Einsatz für das erfindungsgemäße Verfahren verwendet wird. Die Reduzierung des Gehalts an gelösten Gasen in einer Stabilisierungskolonne führt zu einem stabileren Anlagenbetrieb bei der katalytischen Dehydratisierung von Methanol in der Gasphase, da ein Ausgasen in den Rohmethanol-Leitungen oder Zwischenbehältern vermieden wird. Zudem werden potentiell schädliche Gasbestandteile vom Dehydratisierungskatalysator ferngehalten. Bei bereits geringem Anteil an gelösten Gasen im Rohmethanol kann es dagegen vorteilhaft sein, Rohmethanol ohne vorherige Stabilisierung als Einsatz zu verwenden. Der Verzicht auf die Stabilisierungskolonne führt zu einer signifikanten Kosteneinsparung bei den Investitionskosten für die DME-Herstellungsanlage.

Gemäß einer bevorzugten Ausgestaltung der Erfindung wird das in Verfahrensschritt 1 (e) erhaltene Produktgemisch, umfassend Dimethylether, Wasser sowie nicht umgesetztes Methanol, mittels Destillation aufgetrennt. Übliche und an sich bekannte Techniken der Destillation, fraktionierten Destillation oder Rektifikation können verwendet werden. Der nach Auftrennung erhaltene Dimethylether kann nachfolgend als Einsatz zur Herstellung kurzkettiger Olefine, als Brennstoff und/oder Treibstoff oder als Aerosol-Treibgas in Spraydosen verwendet werden.

Die Erfindung betrifft auch ein als Einsatz zur Herstellung von Dimethylether durch katalytische Dehydratisierung in der Gasphase geeignetes Rohmethanol, das dadurch gekennzeichnet ist, dass es einen Gesamtgehalt an Carbonylverbindungen von höchstens 100 Gew.-ppm, bevorzugt von höchstens 50 Gew.-ppm aufweist. Liegen keine weiteren Informationen über die Art der anwesenden Ketone, sondern nur über den Gesamtgehalt an Carbonylverbindungen als Summenparameter vor, ist es sicherer, den niedrigeren Grenzwert für den Gesamtgehalt an Carbonylverbindungen von höchstens 50 Gew.-ppm einzuhalten. Ist andererseits sichergestellt, das nur Aceton als Carbonylverbindung in nachweisbaren Konzentrationen vorhanden ist, kann der höhere Grenzwert für den Gesamtgehalt an Carbonylverbindungen von höchstens 100 Gew.-ppm herangezogen werden.

Weiterbildungen, Vorteile und Anwendungsmöglichkeiten der Erfindung ergeben sich auch aus der nachfolgenden Beschreibung von Ausführungs- und Zahlenbeispielen. Dabei bilden alle beschriebenen Merkmale für sich oder in beliebiger Kombination die Erfindung, unabhängig von ihrer Zusammenfassung in den Ansprüchen oder deren Rückbeziehung.

### Ausführungsbeispiel

Rohmethanol wird in einer Anlage zur katalytischen Methanolsynthese nach dem Niederdruckverfahren erzeugt und einer Stabilisierungskolonne zugeführt. In der Stabilisierungskolonne erfolgt die destillative Auftrennung des Rohmethanols, wobei die Komponenten mit Siedepunkten unterhalb desjenigen des Methanols als Kopfprodukt abgetrennt werden. Das als Sumpfprodukt erhaltene, stabilisierte Rohmethanol wird einem Zwischenbehälter zugeführt. Der Wassergehalt des stabilisierten Rohmethanols beträgt 12 Gew.-%, sein Gesamtgehalt an Carbonylverbindungen liegt bei 50 Gew.-ppm, gerechnet als Aceton, der Acetongehalt bei 30 Gew.-ppm. Aus dem Zwischenbehälter wird das Rohmethanol mittels einer Pumpe entnommen und mittels eines Wärmetauschers im indirekten Wärmetausch gegen die heißen Produktgase des Dehydratisierungsreaktors vorgewärmt bzw. teilverdampft. Die Endverdampfung und das Einstellen der Reaktionstemperatur erfolgt in einem nachgeschalteten Wärmetauscher im indirekten Wärmetausch gegen Hochdruckdampf. Die Einstellung des Reaktionsdruckes erfolgt mit Hilfe eines Druckhalteventils auf der Austrittsseite des Dehydratisierungsreaktors. Der mit stückigem γ-Al₂O₃ -Katalysator gefüllte DME-Reaktor wird mit dem auf die Reaktoreintrittstemperatur von 300 °C gebrachten Rohmethanoldampf beaufschlagt. Die Methanol-Raumgeschwindigkeit beträgt 2,0 kg/(kg·h), der Reaktionsdruck beträgt 16 bar(a). Wegen der vergleichsweise geringen Wärmetönung der Dehydratisierungsreaktion ist der DME-Reaktor als adiabater Festbettreaktor ausgeführt. Im Dehydratisierungsreaktor erfolgt ein Teilumsatz des Rohmethanols zu DME und Wasser entsprechend der Gleichgewichtslage der Dehydratisierungsreaktion in Abhängigkeit von der Temperatur und den Partialdrücken von Methanol und Wasser. Der erzielte Methanol-Umsatz liegt unter diesen Bedingungen zwischen 75 und 82 %; die DME-Selektivität, bezogen auf eingesetztes Methanol, beträgt zwischen 98 und 100 mol-C %.

Das Produktgas wird aus dem Dehydratisierungsreaktor abgeleitet und in einem Wärmetauscher im indirekten Wärmetausch mit dem aus dem Zwischenbehälter entnommenen, kälteren Rohmethanol abgekühlt. Die weitere Abkühlung des Produktgases erfolgt in einem weiteren, wassergekühlten Wärmetauscher, wobei Teilkondensation des Wassers und des nicht umgesetzten Methanols eintritt. Die weitere Produktaufarbeitung erfolgt in an sich bekannter Weise (Ullmann's Encyclopedia of Industrial Chemistry, Sixth Edition, 1998 Electronic Release, Stichwort "Dimethyl Ether", Kapitel 3 "Production") durch zweistufige Destillation, wobei DME in der ersten Destillationstufe als Kopfprodukt erhalten wird. Das erhaltene DME wird in einem nachgeschalteten Kondensator verflüssigt und somit von Leichtsiedern, z. B. Spurengasbestandteilen, getrennt. Auf diese Weise werden DME-Produktreinheiten von > 99,9 % erreicht. Das gasförmige Kopfprodukt des Kondensators wird in einem nachgeschalteten Wäscher mit Rohmethanol als Waschmittel von noch enthaltenen DME-Spuren befreit. Das DME-beladene Rohmethanol wird als Einsatz zum Dehydratisierungsreaktor zurückgeführt. In der zweiten Destillationsstufe wird als Kopfprodukt Methanol erhalten, das ebenfalls als Einsatz zum Dehydratisierungsreaktor zurückgeführt wird. Das als Sumpfprodukt enthaltene Abwasser wird aus dem Verfahren entfernt.

### Zahlenbeispiele

Zur Aufklärung des Grenzwert für den Gesamtgehalt an Carbonylverbindungen für einen sicheren Anlagenbetrieb bei der katalytischen Dehydratisierung von Rohmethanol in der Gasphase wurden mehrere Versuche mit unterschiedlichen Aceton-Konzentrationen in einer Versuchsanlage durchgeführt. Die Versuchsanlage bestand aus einer Rohmethanolversorgung, einem Verdampfer und einem Enderhitzer, einem Festbettreaktor aus Edelstahl mit 27,3 mm Innendurchmesser und einer zweistufigen Kühlung und Abscheidung. Die Abscheidung bestand aus einem Gas/Flüssig-Phasentrenner, als dessen Produkte ein Kondensat und ein Produktgas gewonnen wurden. Analysenproben wurden vom Rohmethanol-Einsatz, vom Kondensat und vom Produktgas genommen, wobei das Produktgas zusätzlich durch eine mit Methanol gefüllte Waschflasche geleitet wurde, um sauerstoffhaltige Spurenbestandteile im Produktgas genauer nachweisen zu können. Verwendet wurde eine gaschromatographische Standard-Analysenmethode für Rohmethanol, mit der Alkohole, Ether, Ester, Ketone und Kohlenwasserstoffe nachgewiesen werden können.

Für alle Versuche wurden die folgenden allegemeinen Versuchsbedingungen eingesetzt:

| | |
|---|---|
| Katalysatoreinwaage: | 150 g |
| Katalysatortyp: | γ-Al₂O₃ als Tabletten (Hersteller: Süd-Chemie) |
| Reaktoreintrittstemperatur: | 300 °C |
| Reaktordruck: | 16 bar(a) |
| Raumgeschwindigkeit: | 2 kg/(kg·h) (gemäß obiger Definition) |

### Beispiele 1 bis 5

Es wurden Versuche mit verschiedenen Aceton-Konzentrationen im Methanol-Einsatz bei ansonsten gleichen identischen Reaktionsbedingungen durchgeführt (Beispiel 1 bis 5), wobei als Referenz ein Versuch ohne Acetonzugabe (Beispiel 1) diente. Die wesentlichen Ergebnisse sind in der folgenden Tabelle zusammengefasst:

| | **Beispiel 1 (Erfindung)** | **Beispiel 2 (Erfindung)** | **Beispiel 3 (Vergleich)** | **Beispiel 4 (Vergleich)** | **Beispiel 5 (Vergleich)** |
|---|---|---|---|---|---|
| Wasseranteil im Einsatz, Gew.-% | 12 | 12 | 12 | 12 | 12 |
| Aceton im Einsatz, Gew.-ppm | 0 | 100 | 2.000 | 10.000 | 100.000 |
| Methanol-Umsatz | 76% | 78% | 76 - 77% | 76% | n.b.* |
| DME-Ausbeute, bezogen auf mol C | 99.9% | 99% | 98 - 99% | 98% | n.b.* |
| unbekannte Komponenten (GC-Peaks) im Kondensat | keine | keine | ca. 70 | ca. 160 | n.b.* |
| unbekannte Komponenten (GC-Peaks) im Produktgas nach Absorption in Methanol | keine | keine | ca. 90 | ca. 200 | n.b.* |
| Verstopfung nach Laufzeit von (maximale Versuchsdauer 50 h) | keine | keine | keine | 1 Tag | <5h |
| Inhaltsstoffe des Feststoffs | - | - | - | HMB | HMB |

| | | | | | |
|---|---|---|---|---|---|
| *n.b. = nicht bestimmt, aufgrund der schnellen Anlagenausfalls konnte keine Komplette Bilanzierung und Analytik der verschiedenen Produktströme durchgeführt werden. | | | | | |

Es zeigte sich, dass bei Konzentrationen ≤ 100 Gew.-ppm Aceton im Einsatz keine Beeinträchtigungen der Umsetzung von Methanol auftraten (Beispiel 1 und Beispiel 2, beide erfindungsgemäß). Bei Konzentrationen von 2000 Gew.-ppm und mehr werden sehr viele unbekannte Produkte gebildet, die in Kondensat und Produktgas nachgewiesen werden (Beispiel 3, Vergleichsbeispiel), allerdings ergab sich nach der maximalen Betriebsdauer von 50 h noch keine Verstopfung in der Versuchsanlage. Wurde die Aceton-Konzentration auf 10000 Gew.-ppm erhöht, so stieg die Zahl der unbekannten Reaktionsprodukte deutlich an und nach ca. einem Tag Versuchsbetrieb wurde eine Verstopfung festgestellt, so dass die Anlage abgestellt werden musste (Beispiel 4, Vergleichsbeispiel). Eine Analyse des die Verstopfung verursachenden Feststoffs zeigte, dass dieser im Wesentlichen aus Hexamethylbenzol (HMB) besteht. Bei einer noch höheren Aceton-Konzentration von 100000 Gew.-ppm (Beispiel 5, Vergleichsbeispiel, 10 Gew.%, in Anlehnung an die oben diskutierten Arbeiten zur Herstellung von HMB) konnte kein regulärer Versuchsbetrieb aufrechterhalten werden, da innerhalb von weniger als 5 h Versuchsbetrieb die Anlage verstopft war. Wiederum bestanden die Ablagerungen aus HMB.

### Beispiel 6 (Vergleichsbeispiel)

In einem weiteren Versuch wurde der Einfluss der MEK-Konzentration untersucht, welches sich gemäß Stand der Technik ähnlich wie das Aceton verhalten und ähnliche Reaktionen eingehen sollte. Der Versuch wurde bei den oben beschriebenen Bedingungen analog Beispiel 1 bis 5 durchgeführt. Die Ergebnisse sind in der folgenden Tabelle zusammengestellt:

| | |
|---|---|
| Wasseranteil im Einsatz, Gew.% | 12 |
| Aceton im Einsatz, Gew.-ppm | 0 |
| MEK im Einsatz, Gew.-ppm | 2000 |
| Gesamtgehalt an Carbonylverbindungen (bezogen auf Massenäquivalente Aceton)^{#)} | 1620 |
| Methanol-Umsatz | 76% |
| DME-Ausbeute, bezogen auf mol C | 98,2 - 99,6% |
| unbekannte Komponenten (GC-Peaks) im Kondensat | ca. 100 |
| unbekannte Komponenten (GC-Peaks) im Produktgas nach Absorption in Methanol | ca. 100 |
| Verstopfung nach Laufzeit von (maximale Versuchsdauer 430 h) | keine |
| Zusammensetzung des Feststoffs | - |

| | |
|---|---|
| #) berechnet über die Beziehung: Massenäquivalente Aceton = Gew.-ppm Carbonylverbindung x Molmasse Aceton / Molmasse Carbonylverbindung | |

Es trat keine Verstopfung der Anlage ein, allerdings zeigt sich auch hier, dass viele neue unbekannte Komponenten durch Nebenreaktionen von MEK und Methanol gebildet werden. Tendenziell werden sogar noch mehr unbekannte Komponenten gebildet als bei einer vergleichbaren Aceton-Konzentration im Rohmethanol-Einsatz (vgl. Beispiel 2); dies lässt sich dadurch begründen, dass MEK im Gegensatz zu Aceton ein unsymmetrisch substituiertes Keton (je eine Methyl- und Ethylgruppe) darstellt, wodurch mehr Kombinationsmöglichkeiten für die Bildung neuer Produkte bestehen.

### Beispiel 7 (Erfindung)

In einem weiteren Versuch in der Anlage unter identischen Bedingungen wurde der Einfluss anderer im Rohmethanol üblicher Verunreinigungen auf den Anlagenbetrieb ermittelt. Die Ergebnisse sind in der folgenden Tabelle enthalten. Die maximale Versuchsdauer mit dieser Einsatzmischung betrug 430 h. Im Unterschied zu den bisherigen Versuchen wurde auch die Temperatur variiert.

| | |
|---|---|
| Wasseranteil im Einsatz, Gew.% | 12 |
| Aceton im Einsatz, Gew.-ppm | 0 |
| MEK im Einsatz, Gew.-ppm | 60 |
| Gesamtgehalt an Carbonylverbindungen (bezogen auf Massenäquivalente Aceton) | 48 |
| Ethanol im Einsatz, Gew.-ppm | 1000 |
| i-Propanol im Einsatz, Gew.-ppm | 280 |
| sec-Butanol im Einsatz, Gew.-ppm | 280 |
| Hexan im Einsatz, Gew.-ppm | 200 |
| Reaktoreintrittstemperatur | 280 - 400°C |
| Methanol-Umsatz | 70-77 |
| DME-Ausbeute bezogen auf mol C | 98,7-99,7 |
| unbekannte Komponenten (GC-Peaks) im Kondensat | 0 |
| unbekannte Komponenten (GC-Peaks) im Produktgas nach Absorption in Methanol | 0 |
| Verstopfung nach Laufzeit von (maximale Versuchsdauer 430 h) | keine |
| Zusammensetzung des Feststoffs | - |

Es ist zu erkennen, dass die Anwesenheit anderer sauerstoffhaltiger Verbindungen, die als Verunreinigungen im Rohmethanol vorkommen, keinen negativen Effekt auf die Dehydratisierung von Rohmethanol hat, sofern der geforderte Grenzwert von 50 Gew.-ppm für den Gesamtgehalt an Carbonylverbindungen eingehalten wird. Dieser Befund gilt auch für die untersuchten deutlich höheren Temperaturen.

### Beispiel 8 (Vergleichsbeispiel)

Um den Effekt der unerwünschten Reaktion von Aceton mit Methanol zu HMB und anderen Komponenten genauer zu untersuchen, wurden 64 g Methanol und 6.4 g Aceton für 20 h bei 230 °C und einem Druck von 20 bar zusammen mit 173 g γ-Al₂O₃ in einem Autoklaven erhitzt. Nach einer Versuchsdauer von 20 h wurde der Versuch beendet und der Katalysator ausgebaut und analysiert. Deutlich waren starke bräunliche Verfärbungen zu erkennen. Analysen des Katalysators zeigten zudem Änderungen der BET-Oberfläche und des Porenvolumens vor und nach der Reaktion, wobei der gebrauchte Katalysator aus Beispiel 7 vor Bestimmung von BET-Oberfläche und Porenvolumen bei 500°C in Inertgas ausgeheizt wurde, um schwerflüchtige organische Komponenten zu desorbieren. Die Untersuchungsergebnisse sind in der nachfolgenden Tabelle zusammengestellt.

| | frischer Katalysator | gebrauchter Katalysator aus Beispiel 7, nach Ausheizen |
|---|---|---|
| BET-Oberfläche, m²/g | 210 | 187 |
| Porenvolumen, m³/g | 0,480 | 0,378 |
| Gewichtsverlust durch Ausheizen bei 500°C | - | 18.3 Gew.% |

Deutlich ist zu erkennen, dass durch die unerwünschten Nebenreaktionen, die bei zu großen Konzentrationen von Aceton im Rohmethanol ablaufen, die BET-Oberfläche und das Porenvolumen deutlich abnahmen. Rechnet man die 18.3 Gew.% an adsorbierten organischen Molekülen mit ein, so sinkt das freie Porenvolumen noch weiter, z.B. bei einer angenommenen Dichte von 1.5 g/cm³ für die Adsorbate um ca. 0.12 m³/g auf nur noch ca. 0.26 m³/g verglichen mit 0.480 cm³/g für den frischen Katalysator. Da es sich bei dem verwendeten Katalysator um einen Vollkatalysator handelt, spielen für die Desaktivierung andere Faktoren wie z. B. Metallbeladung oder Metalldispersion keine Rolle, stattdessen wird die katalytische Aktivität in erster Linie durch die physikalische Zugänglichkeit der katalytisch wirksamen inneren Oberfläche bestimmt. Somit ist aufgrund der beobachteten Reduktion der BET-Oberfläche und des Porenvolumens zu erwarten, dass Laufzeit und Leistung gegenüber einem ordnungsgemäßen Betrieb, also mit einem Einsatz mit einer geringeren Aceton-Konzentration reduziert wird.

Somit hat die Anwesenheit zu hoher Konzentrationen an Carbonylverbindungen nicht nur eine Beeinträchtigung des Verfahrens durch Bildung von Ablagerungen z. B. in Rohrleitungen zur Folge, die jeweils zu einem unerwünschten Stillstand der Anlage führen würden und die Anlagenverfügbarkeit reduzieren, sondern sie führen auch einer Schädigung des Katalysators und bewirken somit geringere Methanol-Umsätze und DME-Ausbeuten.

### Gewerbliche Anwendbarkeit

Mit der Erfindung wird somit ein verbessertes Verfahren zur Herstellung von Dimethylether zur Verfügung gestellt, das sich aufgrund des Einsatzes von Rohmethanol zur Dehydratisierung durch ökonomische Vorteile gegenüber einem Reinmethanol-basierten Verfahren auszeichnet. Mindestens eine Destillationsstufe zur Rohmethanol-Aufarbeitung wird auf diese Weise eingespart. Die Vermeidung der Abdestillation großer Mengen an Methanol als Leichtsieder in der Reinmethanol-Kolonne reduziert den Energieverbrauch des Verfahrens signifikant. Der Einsatz von Rohmethanol zur Dehydratisierung ist unproblematisch, wenn die in den Ansprüchen angegebenen Grenzwerte für den Gesamtgehalt an Carbonylverbindungen eingehalten werden. Es wird ein DME-Produkt erhalten, das trotz des Einsatzes von Rohmethanol besonders arm an störenden Verunreinigungen ist.

## Patentansprüche

1. Verfahren zur Herstellung von Dimethylether durch katalytische Dehydratisierung von Rohmethanol als Einsatz in der Gasphase, umfassend folgende Verfahrensschritte:
(a) Bereitstellen von Rohmethanol aus der Niederdruck-Methanolsynthese,
(b) Verdampfen des Rohmethanols und Einstellen einer Reaktionstemperatur und eines Reaktionsdruckes,
(c) Beaufschlagen eines mit Dehydratisierungskatalysator gefüllten Reaktors mit dem verdampften Rohmethanol mit definierter Raumgeschwindigkeit,
(d) Ableiten eines gasförmigen Produktgemisches, umfassend Dimethylether, nicht umgesetztes Methanol und Wasser,
(e) Abkühlen, Teilkondensation und Auftrennung des gasförmigen Produktgemisches, wobei als Produkte gasförmiger Dimethylether sowie flüssiges Wasser und Methanol erhalten werden, wobei das Methanol zum Verfahrensschritt 1 (a) zurückgeführt wird,
**dadurch gekennzeichnet, dass** das als Einsatz verwendete Rohmethanol einen Gesamtgehalt an Carbonylverbindungen von höchstens 100 Gew.-ppm, bevorzugt von höchstens 50 Gew.-ppm aufweist, gerechnet als Massenäquivalente Aceton.

2. Verfahren nach Anspruch 1, **dadurch gekennzeichnet, dass** als Reaktor ein Festbettreaktor verwendet wird.

3. Verfahren nach Anspruch 1 oder 3, **dadurch gekennzeichnet, dass** als Katalysator γ-Al₂O₃ verwendet wird.

4. Verfahren nach einem der vorgenannten Ansprüche, **dadurch gekennzeichnet, dass** die Reaktionstemperatur zwischen 200 und 500 °C, bevorzugt zwischen 250 und 450 °C liegt.

5. Verfahren nach einem der vorgenannten Ansprüche, **dadurch gekennzeichnet, dass** der Reaktionsdruck zwischen 1 und 100 bar(a), bevorzugt zwischen 1 und 30 bar(a) liegt.

6. Verfahren nach einem der vorgenannten Ansprüche, **dadurch gekennzeichnet, dass** die Raumgeschwindigkeit zwischen 1 und 8 kg/(kg·h), bevorzugt zwischen 1 und 6 kg/(kg·h) liegt.

7. Verfahren nach einem der vorgenannten Ansprüche, **dadurch gekennzeichnet, dass** man stabilisiertes Rohmethanol als Einsatz verwendet.

8. Verfahren nach einem der vorgenannten Ansprüche, **dadurch gekennzeichnet, dass** man Rohmethanol ohne vorherige Stabilisierung als Einsatz verwendet.

9. Verfahren nach einem der vorgenannten Ansprüche, **dadurch gekennzeichnet, dass** die Auftrennung des in Verfahrensschritt 1 (e) erhaltenen Produktgemisches mittels Destillation erfolgt.

## Claims

1. A method for producing dimethyl ether by catalytic dehydration of crude methanol as feedstock in the gas phase, comprising the following method steps:
(a) providing crude methanol from the low-pressure methanol synthesis,
(b) evaporating the crude methanol and setting a reaction temperature and a reaction pressure,
(c) charging a reactor filled with dehydration catalyst with the evaporated crude methanol at a defined space velocity,
(d) discharging a gaseous product mixture, comprising dimethyl ether, non-converted methanol and water,
(e) cooling, partial condensation and separation of the gaseous product mixture, wherein gaseous dimethyl ether as well as liquid water and methanol are obtained as products, the methanol being recirculated to method step 1(a),
**characterized in that** the crude methanol used as feedstock has a total content of carbonyl compounds of not more than 100 wt-ppm, preferably not more than 50 wt-ppm, calculated as mass equivalents of acetone.

2. The method according to claim 1, **characterized in that** a fixed-bed reactor is used as reactor.

3. The method according to claim 1 or 3, **characterized in that** γ-Al₂O₃ is used as catalyst.

4. The method according to any of the preceding claims, **characterized in that** the reaction temperature lies between 200 and 500 °C, preferably between 250 and 450 °C.

5. The method according to any of the preceding claims, **characterized in that** the reaction pressure lies between 1 and 100 bar(a), preferably between 1 and 30 bar(a).

6. The method according to any of the preceding claims, **characterized in that** the space velocity lies between 1 and 8 kg/(kg·h), preferably between 1 and 6 kg/(kg·h).

7. The method according to any of the preceding claims, **characterized in that** stabilized crude methanol is used as feedstock.

8. The method according to any of the preceding claims, **characterized in that** crude methanol is used as feedstock without previous stabilization.

9. The method according to any of the preceding claims, **characterized in that** the separation of the product mixture obtained in method step 1(e) is effected by means of distillation.

## Revendications

1. Procédé de préparation d'oxyde de diméthyle, par déshydratation catalytique de méthanol brut comme charge en phase gazeuse, comprenant les stades de procédé suivantes :
(a) on se procure du méthanol brut provenant de la synthèse du méthanol sous basse pression,
(b) on évapore le méthanol brut et on établit une température de réaction et une pression de réaction,
(c) on charge un réacteur garni de catalyseur de déshydratation du méthanol brut évaporé à une vitesse spatiale définie,
(d) on évacue un mélange gazeux de produits comprenant de l'oxyde de diméthyle, du méthanol inaltéré et de l'eau,
(e) on refroidit, on condense partiellement et on sépare le mélange gazeux de produits, en obtenant comme produits de l'oxyde de diméthyle gazeux, ainsi que de l'eau liquide et du méthanol, le méthanol étant retourné au stade 1 (a) du procédé,
**caractérisé en ce que** le méthanol brut utilisé comme charge a une teneur totale en composés carbonyle de 100 parties par million en poids au plus, de préférence de 50 parties par million en poids au plus, calculée en équivalent massique d'acétone.

2. Procédé suivant la revendication 1, **caractérisé en ce que** l'on utilise comme réacteur un réacteur à lit fixe.

3. Procédé suivant la revendication 1 ou 2, **caractérisé en ce que** l'on utilise comme catalyseur de l'Al₂O₃ γ.

4. Procédé suivant l'une des revendications précédentes, **caractérisé en ce que** la température de réaction est comprise entre 200 et 500°C, de préférence entre 250 et 450°C.

5. Procédé suivant l'une des revendications précédentes, **caractérisé en ce que** la pression de réaction est comprise entre 1 et 100 bar (absolus), de préférence entre 1 et 30 bar (absolus).

6. Procédé suivant l'une des revendications précédentes, **caractérisé en ce que** la vitesse spatiale est comprise entre 1 et 8 kg/(kgh), de préférence entre 1 et 6 kg/kgh).

7. Procédé suivant l'une des revendications précédentes, **caractérisé en ce que** l'on utilise comme charge du méthanol brut stabilisé.

8. Procédé suivant l'une des revendications précédentes, **caractérisé en ce que** l'on utilise comme charge du méthanol brut sans stabilisation préalable.

9. Procédé suivant l'une des revendications précédentes, **caractérisé en ce que** l'on effectue la séparation du mélange de produits obtenu au stade 1 (e) du procédé au moyen d'une distillation.
